(19) Europäisches Patentamt
European Patent Office
Office européen des brevets

(11) Veröffentlichungsnummer: **0 324 390**
**A2**

(12) # EUROPÄISCHE PATENTANMELDUNG

(21) Anmeldenummer: **89100220.6**

(22) Anmeldetag: **07.01.89**

(51) Int. Cl.4: **C07D 493/10 , A61K 31/35 , //(C07D493/10,311:00,307:00)**

Patentansprüche für folgende Vertragsstaaten: ES + GR.

Ein Antrag gemäss Regel 88 EPÜ auf Berichtigung der Beschreibung und der Ansprüche liegt vor. Über diesen Antrag wird im Laufe des Verfahrens vor der Prüfungsabteilung eine Entscheidung getroffen werden (Richtlinien für die Prüfung im EPA, A-V, 2.2).

(30) Priorität: **12.01.88 DE 3800598**
**02.09.88 DE 3829780**

(43) Veröffentlichungstag der Anmeldung:
**19.07.89 Patentblatt 89/29**

(84) Benannte Vertragsstaaten:
**AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Hammann, Peter, Dr.**
**Mörikestrasse 6**
**D-6233 Kelkheim (Taunus)(DE)**
Erfinder: **Grabley, Susanne, Dr.**
**Hölderlinstrasse 7**
**D-6240 Königstein/Taunus(DE)**
Erfinder: **Voelskow, Hartmut, Dr.**
**Akazienstrasse 22**
**D-6234 Hattersheim 3(DE)**
Erfinder: **Winkler, Irvin, Dr.**
**In den Eichen 40**
**D-6237 Liederbach(DE)**
Erfinder: **Seibert, Gerhard, Prof. Dr.**
**Glaeserweg 21**
**D-6100 Darmstadt(DE)**
Erfinder: **Zeeck, Axel, Prof. Dr.**
**Brüder-Grimm-Allee 22**
**D-3400 Göttingen(DE)**
Erfinder: **Steinhoff, Ilsemarie, Dr.**
**An den Matten 6**
**D-3401 Mengershausen(DE)**

(54) **Nigericinderivate, Verfahren zu deren Herstellung, diese enthaltende Mittel und Verwendung derselben sowie die Verwendung von Nigericin als antiviral wirksame Substanz.**

㊼ Die Erfindung betrifft Nigericinderivate der Formel I

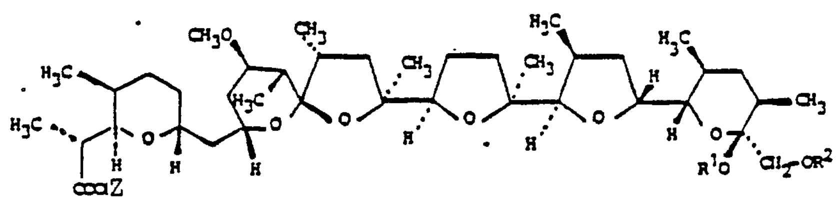

(I)

in der Z, $R^1$ und $R^2$ die angegebenen Bedeutungen haben. Die Verbindungen der Formel I zeigen sowohl antibakterielle als auch antivirale Wirksamkeit.

EP 0 324 390 A2

## Nigericinderivate, Verfahren zu deren Herstellung, diese enthaltende Mittel und Verwendung derselben sowie die Verwendung von Nigericin als antiviral wirksame Substanz

Der Polyether Nigericin, der durch Kultivierung von Streptomyces hygroscopicus erhalten werden kann, ist u. a. beschrieben in J. Berger et al., Am. Chem. Soc. 73, 5295 (1951)

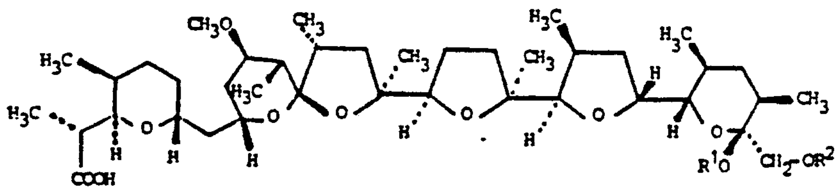

und wurde bislang als Antibiotikum eingesetzt.

Einige Derivate des Nigericins sind ebenfalls bekannt (JP 72 01,288 und Tokuo Kubota et al., J. Chem. Soc. (C) 1970, 695), auch sie wurden bislang als Antibiotika eingesetzt.

Es wurde nun gefunden, daß Nigericin und dessen Derivate ausgezeichnete antiviral wirksame Substanzen sind.

Die Erfindung betrifft nun

1. Nigericinderivate der Formel I

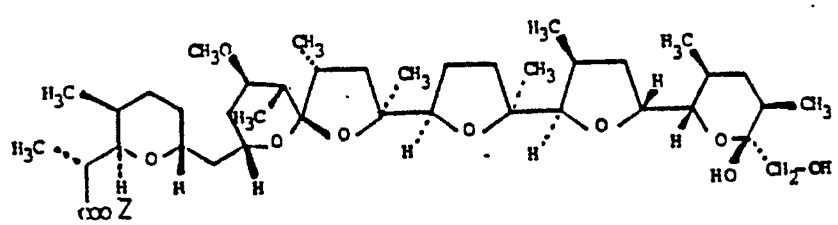

(I)

in der Z H, Na, K, Li, Mg, Ca oder $NH_4$ bedeutet und
$R^1$ Wasserstoff oder $C_1$-$C_8$-Alkyl bedeutet und
$R^2$ Wasserstoff bedeutet oder einen Rest der Formel
$-\overset{O}{\overset{\|}{C}}-R^3$, $-\overset{O}{\overset{\|}{C}}-NH-R^3$ oder $-\overset{O}{\overset{\|}{C}}-O-R^3$ darstellt, in der $R^3$ $C_1$-$C_{26}$-Alkyl, $C_2$-$C_{15}$-Alkenyl oder $C_3$-$C_9$-Cycloalkyl bedeutet, wobei diese Alkyl, Alkenyl-und Cycloalkylreste gegebenenfalls halogen-, nitro-, cyano- carboxyl-, $C_1$-$C_4$-alkoxy-, thienyl-, phenyloxy- oder phenylsubstituiert sind, wobei die genannten Phenylreste ihrerseits mit Halogen, Nitro, Cyano, $C_1$-$C_4$-Alkyl oder $C_1$-$C_4$-Alkoxy substituiert sein können, oder in der
$R^3$ Phenyl, Furyl oder Thienyl bedeutet, wobei diese Phenyl-, Furyl- oder Thienyl-Reste mit Halogen, Nitro, Cyano, Carboxyl-, $C_1$-$C_4$-Alkyl oder $C_1$-$C_4$-Alkoxy substituiert sein können
oder worin
$R^2$ einen Sulfonsäureester der Formel $-SO_2R^4$ darstellt, worin
$R^4$ Hydroxy, $C_1$-$C_6$-Alkyl, Phenyl oder p-Toloyl bedeutet
oder worin
$R^1$ und $R^2$ zusammen einen Rest der Formel II darstellen

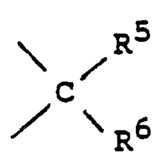

(II)

in der
$R^5$ und $R^6$ gleich oder verschieden sind und Wasserstoff oder $C_1$-$C_{10}$-Alkyl bedeuten oder wobei

$R^5$ und $R^6$ eine Alkylenkette darstellen, die zusammen mit dem sie tragenden C-Atom einen 5-, 6- oder 7-gliedrigen Ring bildet,
sowie die physiologisch verträglichen Salze dieser Verbindungen, ausgenommen die Verbindungen der Formel I, in der $R^1$ und $R^2$ gleichzeitig Wasserstoff bedeuten (Nigericin) und solche, in der $R^1$ Wasserstoff und $R^2$ Acetyl oder Benzoyl bedeuten und das Natriumsalz der Verbindung der Formel I, in der $R^1$ und $R^2$ zusammen einen Rest der Formel II darstellen, mit $R^5 = R^6 = CH_3$.

2. Die vorgenannten Verbindungen zur Anwendung als Arzneimittel.

3. Die vorgenannten Verbindungen zur Anwendung als antibakteriell wirkende Arzneimittel.

4. Die vorgenannten Verbindungen, jedoch einschließlich Nigericin und der Nigericinderivate der Formel I, in der $R^1$ Wasserstoff und $R^2$ Acetyl oder Benzoyl bedeuten und das Natriumsalz der Verbindung der Formel I, in der $R^1$ und $R^2$ zusammen einen Rest der Formel II darstellen, mit $R^5 = R^6 = CH_3$, zur Anwendung als antiviral wirksame Arzneimittel.

5. Ein Verfahren zur Herstellung der Verbindungen der Formel I, wobei man
a) Nigericin (Formel I : $R^1 = R^2 = H$) oder ein $R^1$-Derivat umsetzt mit einer Verbindung der Formel III oder IV

$X-\overset{\overset{O}{\|}}{C}-R^3$ (III)

$X-\overset{\overset{O}{\|}}{C}-O-R^3$ (IV)

worin
X Chlor, Brom oder ein Anhydrid bedeutet und
$R^3$ die oben zu Formel I angegebenen Bedeutungen hat,
oder wobei man
b) Nigericin (Formel I : $R^1 = R^2 = H$) oder ein $R^1$-Derivat umsetzt mit einem Isocyanat der Formel V
$O = C = N - R^3$ (V)
worin $R^3$ die oben zu Formel I angegebenen Bedeutungen hat,
oder wobei man
c) Nigericin (Formel I : $R^1 = R^2 = H$) oder ein $R^1$-Derivat umsetzt mit einem $SO_3$/Pyridin-Komplex oder mit einer Verbindung der Formel $Y-SO_2-R^4$, wobei Y Chlor oder Brom bedeutet und $R^4$ bis auf Wasserstoff, die oben zu Formel I angegebenen Bedeutungen hat,
oder wobei man
d) Nigericin (Formel I : $R^1 = R^2 = H$) oder ein $R^2$-Derivat in Gegenwart einer Lewis-Säure umsetzt mit einem Alkohol der Formel VI
$R^1 - OH$ (VI)
worin $R^1$ bis auf Wasserstoff die oben zu Formel I angegebenen Bedeutungen hat,
oder wobei man
e) Nigericin (Formel I : $R^1 = R^2 = H$) in Gegenwart eines Katalysators umsetzt mit einer Carbonylverbindung der Formel VII

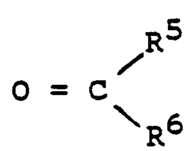

(VII)

worin $R^5$ und $R^6$ die oben zu Formel I angegebenen Bedeutungen haben,
und wobei man gegebenenfalls anschließend die Verbindungen der Formel I in ihr physiologisch verträgliches Salz überführt.

Unter Halogenen werden Fluor, Chlor, Brom und Jod verstanden, insbesondere Chlor und Brom.
Unter einem $R^1$- bzw. $R^2$-Derivat werden solche Verbindungen der Formel I verstanden, bei denen $R^2$ (= $R^1$-Derivat) bzw. $R^1$ (= $R^2$-Derivat) Wasserstoff bedeutet und der jeweils andere Rest von Wasserstoff verschieden ist.
Unter Lewis-Säuren werden hier Kupfer-, Eisen- oder Lithiumhalogenide wie Kupferchlorid, Eisen(III)-chlorid oder Lithiumbromid, insbesondere Lithiumbromid, verstanden.

Alkyl- und Alkenylgruppen mit mehr als 2 Kohlenstoffatomen können sowohl geradkettig als auch verzweigt sein.

Die genannten Alkyl-, Alkenyl-, Cycloalkyl-, Phenyl-, Furyl- und Thienylgruppen können sowohl einfach

als auch mehrfach substituiert sein.

Im folgenden werden die Verfahren a) bis e), die es ermöglichen, die unterschiedlich substituierten Nigericinderivate herzustellen, näher beschrieben.

Mit Hilfe der Verfahrensvarianten a), b) und c) lassen sich unterschiedliche Substituenten $R^2$ in das Molekül einführen. Mit Hilfe der Verfahrensvarianten d) läßt sich der Substituent $R^1$ in das Molekül einführen. Verfahrensvariante e) dient zur Herstellung solcher Nigricinderivate, in denen $R^1$ und $R^2$ zusammen einen Rest der Formel II darstellen.

Die Reihenfolge der Derivatisierung ist im allgemeinen unkritisch, d. h. es kann sowohl zunächst der Substituent $R^2$ und gegebenenfalls anschließend der Substituent $R^1$ in das Molekül eingeführt werden, als auch umgekehrt, also zunächst $R^1$ und dann $R^2$.

Bei der Verfahrensvariante a) geht man am besten so vor, daß man Nigericin oder ein $R^1$-Derivat in äquimolaren Mengen oder in bis zu einem 50-fachen Überschuß, gegebenenfalls in einem inerten, aprotischen Lösungsmittel wie Chloroform, Methylenchlorid, Tetrahydrofuran (THF), Essigester oder Dioxan, mit einer Verbindung der Formel III oder IV - insbesondere den Anhydriden - bis zur Beendigung der Reaktion umsetzt, gegebenenfalls in Gegenwart einer Base, vorzugsweise Pyridin oder DMAP (Dimethylaminopyridin).

Die Reaktionstemperaturen liegen dabei zwischen - 70° C und +100° C, vorzugsweise bei Verwendung eines Lösungsmittels zwischen dem Festpunkt und dem Siedepunkt des Lösungsmittels, insbesondere zwischen - 70° C und +40° C. Die Reaktionszeiten betragen 1 bis 180 Stunden, bevorzugt 1 bis 48 Stunden. Die Beendigung der Reaktion kann beispielsweise mittels Dünnschichtchromatographie bestimmt werden (DC-Kontrolle).

Das als Ausgangssubstanz benötigte Nigericin kann beispielsweise nach dem von J. Berger (loc. cit. Am. Chem. Soc.) beschriebenen Verfahren hergestellt werden. Es kann aber auch gemäß dem in der deutschen Patentanmeldung P 3700325.9 vorgeschlagenen Verfahren hergestellt werden, wobei Nigericin neben Amycin bei der Kultivierung von Streptomyces parvulus DSM 3816 entsteht. Das Nigericin kann aus dem Mycel mittels Hexan extrahiert und nach Aufkonzentrierung auskristallisiert werden.

Die Ausgangsverbindungen für die Verfahrensvariante a), die Verbindungen der Formel III oder IV sind, sofern nicht käuflich, auf einfache Weise nach literaturbekannten Verfahren herstellbar. Beispielsweise erhält man die Säurechloride durch Umsetzung der entsprechenden Carbonsäure mit Thionylchlorid, $PCl_3$ oder $PCl_5$. Solche Verfahren sind beispielsweise beschrieben in Gattermann/Wieland, "Die Praxis des Organischen Chemikers", 43. Auflage, Walter de Gruyter, Berlin, New York 1982, S. 303 ff. (Zur Herstellung der Anhydride siehe beispielsweise: ORGANIKUM, Organisch-Chemisches Grundpraktikum, 15. Auflage (1976), VEB Deutscher Verlag der Wissenschaften, Berlin, Methodenregister S. 824: Carbonsäureanhydride).

Bei der Verfahrensvarianten b) geht man am besten so vor, daß man das Nigericin oder ein $R^1$-Derivat in äquimolaren Mengen oder in einem bis zu 50-fachen Überschuß umsetzt, mit einer Verbindung der Formel V.

Gegebenenfalls kann auch diese Umsetzung unter Basenzusatz erfolgen. Als Basen kommen beispielsweise Triethylamin, Pyridin oder Lutidin in Frage. Eine Variante des Verfahrens b) besteht darin, daß man in einem geeigneten, vorzugsweise inerten Lösungsmittel wie Chloroform, Methylenchlorid, THF, Essigester oder Dioxan arbeitet. Auch hier kann der Überschuß der oben aufgeführten Verbindungen bis zur 50-fachen Menge betragen.

Die Reaktionstemperaturen liegen dabei zwischen -70° C und +100° C, vorzugsweise bei Verwendung eines Lösungsmittels zwischen dem Festpunkt und dem Siedepunkt des Lösungsmittels, insbesondere zwischen - 70° C und +40° C. Die Reaktionszeiten betragen 1 bis 180 Stunden, bevorzugt 1 bis 49 Stunden. Die Beendigung der Reaktion kann beispielsweise mittels DC-Kontrolle bestimmt werden.

Die Ausgangsverbindungen für die Verfahrensvariante b) sind, sofern nicht käuflich, auf einfache Weise nach literaturbekannten Verfahren herstellbar (Isocyanate: Houben-Weyl, 4. Auflage, Georg Thieme Verlag Stuttgart (1983), Band E4).

Bei der Verfahrensvariante c) geht man am besten analog der Verfahrensvarianten b) vor. Der $SO_3$/Pyridin-Komplex ist käuflich erhältlich. Die Aryl- bzw. Alkylsulfonsäurehalogenide der Formel $Y\text{-}SO_2R^4$ erhält man beispielsweise durch radikalische Umsetzung von Alkanen mit Chlor und $SO_2$ oder durch Halogenierung von Aromaten mit Halogensulfonsäure $Y\text{-}SO_3H$.

Bei der Verfahrensvariante d) geht man am besten so vor, daß man Nigericin oder ein entsprechendes $R^2$-Derivat mit einem Überschuß an einem Alkohol der Formel $R^1$-OH in Gegenwart einer Lewissäure bis zur Beendigung der Reaktion umsetzt. Eine Variante des Verfahrens besteht darin, daß man in einem geeigneten Lösungmittel wie Chloroform, Methylenchlorid, THF, Essigester oder Dioxan arbeitet. Als Lewissäure eignen sich beispielsweise Kupfer-, Eisen- oder Lithium-Halogenide, insbesondere $CuCl_2$, $FeCl_3$ oder LiBr. Ganz besonders bevorzugt ist die Verwendung von Lithiumbromid.

Die Konzentration der Lewissäure - bezogen auf Nigericin oder das Nigericinderivat - beträgt 0,1 bis 50 Gew-%, bevorzugt 0,5 bis 20 Gew.-%. Die Reaktionstemperaturen liegen dabei zwischen -40° C und +100° C, insbesondere zwischen 0° C und 30° C, vorzugsweise bei Verwendung eines Lösungsmittels zwischen dem Festpunkt und dem Siedepunkt des Lösungsmittels, insbesondere zwischen 0° C und 30° C. Die Reaktionszeiten betragen 1 bis 180 Minuten, bevorzugt 5 bis 60 Minuten. Die Beendigung der Reaktion kann beispielsweise mittels Dünnschichtchromatographie bestimmt werden.

Bei der Verfahrensvariante e) geht man am besten so vor, daß man Nigericin im Überschuß mit einer Verbindung der Formel VII bis zur Beendigung der Reaktion umsetzt, gegebenenfalls in Gegenwart eines Katalysators wie $ZnCl_2$, $ZnBr_2$, $FeCl_3$, $CuSO_4$ oder $CuCl_2$. Die Konzentration dieses Katalysators - bezogen auf Nigericin - beträgt vorzugsweise 0,1 bis 50 %. Eine Variante des Verfahrens besteht darin, daß man in einem geeigneten aprotischen Lösungsmittel wie Chloroform, Methylenchlorid, THF, Essigester oder Dioxan arbeitet. Auch hier kann ein Überschuß an VII angewandt werden.

Die Reaktionstemperaturen liegen dabei zwischen -20° C und +100° C, vorzugsweise bei Verwendung eines Lösungsmittels zwischen Raumtemperatur und dem Siedepunkt des Lösungsmittels, insbesondere zwischen 20° C und 100° C. Die Reaktionszeiten betragen 1 bis 50 Stunden, bevorzugt 1 bis 12 Stunden. Die Beendigung der Reaktion kann beispielsweise mittels DC-Kontrolle bestimmt werden.

Die Ausgangsverbindungen für die Verfahrensvariante e), die Verbindungen der Formel VII sind, sofern nicht käuflich, auf einfache Wiese nach literaturbekannten Verfahren herstellbar. Beispielsweise erhält man Aldehyde der Formel VII durch Oxidation von primären Alkoholen oder durch Reduktion von Carboxylderivaten, beispielsweise mit komplexen Hydriden oder durch Reduktion von Carbonsäurechloriden (weitere Beispiele zur Synthese von Aldehyden bzw. Ketonen finden sich in Organikum, Organisch Chemisches Praktikum, 15. Auflage, VEB Deutscher Verlag der Wissenschaften, Berlin 1976, Methodenregister, Aldehyde, Ketone, S. 819 ff.).

Die Herstellung der physiologisch verträglichen Salze erfolgt nach an sich bekannten Methoden durch Umsatz mit anorganischen oder organischen Basen. Zur Salzbildung sind insbesondere geeignet Alkali- und Erdalkalihyroxyde, -carbonate oder -bicarbonate sowie physiologisch verträgliche organische Verbindungen, die eine primäre, sekundäre oder tertiäre Aminogruppe tragen.

Die Reinigung, Isolierung und Aufarbeitung der Substanzen erfolgt nach den üblichen Methoden; beispielsweise können die Reaktionsprodukte durch Chromatographie an polaren Trägermaterialien wie Kieselgel oder ®Sephadex LH 20 mit Lösungsmitteln wie niederen Alkanolen wie Methanol oder Chloroform oder Essigester oder Methanol/Chloroform-Mischungen, aber auch extraktive Methoden wie flüssig/flüssig-Extraktion oder fest/flüssig-Extraktion oder durch Kristallisation gereinigt werden.

Nigericin selbst sowie seine Derivate zeigen ausgezeichnete antivirale Wirkung. Diese antivirale Aktivität wurde in Zellkulturen, die mit Testviren infiziert wurden, getestet. Ebenso zeigen die Derivate eine antibakterielle Wirkung.

Die erfindungsgemäßen Verbindungen sind aufgrund ihrer pharmakologischen Eigenschaften für die Behandlung von bakteriellen Erkrankungen und Viruserkrankungen, die hervorgerufen werden beispielsweise durch HSV I, II (Herpes simplex I- oder II-Virus) oder auch Picorna- und Retroviren, wie HIV (Human Immunodeficiency Virus) geeignet.

Die Erfindung betrifft daher weiter die Anwendung der erfindungsgemäßen Verbindungen der Formel I sowie deren physiologisch verträgliche Salze bei der Behandlung und Prophylaxe von bakteriellen Erkrankungen oder von Herpes-Virus-, Picorna- und Retroviren-Erkrankungen.

Die neuen Verbindungen können entweder allein oder mit physiologisch verträglichen Hilfs- oder Trägerstoffen vermischt als Arzneimittel angewandt werden. Sie können zu diesem Zweck oral in Dosen von 0,01 - 5,0 mg/kg/Tag, vorzugsweise 0,01 - 1,0 mg/kg/Tag oder parenteral subkutan in Dosen von 0,001 - 2,5 mg/kg/Tag, vorzugsweise 0,001 -1,0 mg/kg/Tag, insbesondere 0,005 - 0,2 mg/kg/Tag, appliziert werden. Besonders bevorzugt ist die topische Anwendung, wobei die Wirkstoffkonzentration in den Salben 0,001 - 1 %, bevorzugt 0,01 - 0,1 %, beträgt. Die Dosierung kann in schweren Fällen auch erhöht werden. In vielen Fällen genügen jedoch auch geringere Dosen.

Die Erfindung umfaßt weiterhin die Verwendung der erfindungsgemäßen Verbindungen bei der Herstellung von Arzneimitteln, die zur Behandlung und Prophylaxe der vorstehend genannten Krankheiten eingesetzt werden.

Ein weiterer Gegenstand der Erfindung sind Arzneimittel, die ein oder mehrere erfindungsgemäße Verbindungen der Formel I enthalten.

Die Arzneimittel werden nach an sich bekannten, dem Fachmann geläufigen Verfahren hergestellt. Als Arzneimittel werden die erfindungsgemäßen pharmakologisch wirksamen Verbindungen (= Wirkstoff) entweder als solche oder vorzugsweise in Kombination mit geeigneten pharmazeutischen Hilfs- oder Trägerstoffen in Form von Tabletten, Dragees, Kalseln, Suppositorien, Emulsionen, Suspensionen oder Lösungen

eingesetzt, wobei der Wirkstoffgehalt bis etwa 95 %, vorteilhafterweise zwischen 10 und 75 % beträgt. Bei der topischen Anwendung reichen schon Wirkstoffkonzentrationen von 0,001 - 1 %, bevorzugt 0,01 -0,1 %, aus.

Geeignete Hilfs- bzw. Trägerstoffe für die gewünschte Arzneimittelformulierung sind beispielsweise neben Lösemitteln, Gelbildnern, Suppositoriengrundlagen, Tabletten-Hilfsstoffen und anderen Wirkstoffträgern auch Antioxidantien, Dispergiermittel, Emulgatoren, Entschäumer, Geschmackskorrigentien, Konservierungsmittel, Lösungsvermittler oder Farbstoffe.

Die Wirkstoffe können oral, parenteral (subkutan), topisch oder rectal appliziert werden, wobei die topische Applikation bevorzugt ist.
Die aktiven Verbindungen werden mit den dafür geeigneten Zusatzstoffen wie Trägerstoffen, Stabilisatoren oder inerten Verdünnungsmitteln vermischt und durch die üblichen Methoden in geeignete Darreichungsformen gebracht, wie Tabletten, Dragees, Steckkapseln, wäßrige alkoholische oder ölige Suspensionen oder wäßrige oder ölige Lösungen, Cremes oder Salben. Als inerte Trägerstoffe können z. B. Gummi arabicum, Magnesia, Magnesiumcarbonat, Kaliumphosphat, Milchzucker, Glukose oder Stärke, insbesondere Maisstärke, verwendet werden. Dabei kann die Zubereitung sowohl als Trocken- als auch als Feuchtgranulat erfolgen. Als ölige Trägerstoffe oder Lösemittel kommen beispielsweise pflanzliche oder tierische Öle in Betracht, wie Sonnenblumenöl oder Lebertran.

Zur subkutanen oder intravenösen Applikation werden die aktiven Verbindungen gewünschtenfalls mit den dafür geeigneten Substanzen wie Lösungsvermittler, Emulgatoren oder weiteren Hilfsstoffen in Lösung, Suspension oder Emulsion gebracht. Als Lösungsmittel kommen z. B. in Frage physiologische Kochsalzlösung oder Alkohole, z. B. Ethanol, Propanol, Glycerin, daneben auch Zuckerlösungen wie Glucose- oder Mannitlösungen, oder auch eine Mischung aus den verschiedenen genannten Lösungsmitteln.

Nachfolgend ist die Erfindung an Hand von Beispielen näher erläutert.

## Allgemeine Verfahrensvorschriften

### Verfahren 1

725 mg (1 mmol) Nigericin werden in 10 ml Pyridin gelöst und mit 1,5 mmol Anhydrid in Gegenwart von 10 mg Dimethylaminopyridin für 4 - 24 h umgesetzt. Nach der Zugabe von 40 ml Wasser wird weitere 20 min bei Raumtemperatur gerührt. Die wäßrige Phase wird mit Ethylacetat ( 3 x 20 ml) extrahiert und die organische Phase wird mit 2 x 10 ml 0,1 N HCl und 2 x 10 ml Wasser gewaschen. Nach dem Trocknen über Natriumsulfat wird im Vakuum einrotiert. Chromatographie an 250 g Kieselgel mit Chloroform/Methanol von 30 : 1 auf 9 : 1 ergibt die Ester.

### Verfahren 2

725 mg (1 mmol) Nigericin werden in 1,5 mmol Säurechlorid für 1 - 24 h in Pyridin (10 ml) gerührt. Nach der Zugabe von Wasser werden weitere 20 min bei Raumtemperatur gerührt. Die wäßrige Phase wird mit Ethylacetat ( 3 x 20 ml) extrahiert und die organische Phase wird mit 2 x 10 ml 0,1 N HCl und 2 x 10 ml Wasser gewaschen. Nach dem Trocknen über Natriumsulfat wird im Vakuum einrotiert.
Chromatographie an 250 g Kieselgel mit Chloroform/Methanol von 30 : 1 auf 9 : 1 ergibt die Ester.

### Verfahren 3

725 mg (1 mmol) Nigericin werden in 20 ml Chloroform gelöst und mit 1,5 mmol Isocyanat für 24 h bei Raumtemperatur gerührt. Nach der Zugabe von Wasser (50 ml) wird mit 2 - 20 ml Chloroform extrahiert. Nach dem Trocknen über Natriumsulfat wird bis zu einem Sirup einrotiert. Chromatographie an 250 g Keiselgel mit Chloroform/Methanol von 30 : 1 auf 9 : 1 ergibt die Carbaminsäurederivate.

### Verfahren 4

725 mg (1 mmol) Nigericin werden in 10 ml Chloroform mit 300 mg $SO_3$·Pyridin-Komplex für 20 h

gerührt. Die organische Phase wird mit 0,1 N HCl gewaschen und anschließend über Natriumsulfat getrocknet. Chromatographie an Keiselgel mit Chloroform/Methanol von 30 : 1 auf 9 : 1 ergibt das Produkt.

**Verfahren 5**

725 mg (1 mmol) Nigericin werden in 10 ml Alkohol gelöst und mit 100 mg Lithiumbromid für 2 h unter Rückfluß erhitzt. Anschließend wird bis zur Trockne eingedampft. Der Rückstand wird in 20 ml Ethylacetat aufgenommen und mit Wasser (2 x 20 ml) extrahiert. Nach dem Einrotieren wird das Ketal als fester Rückstand erhalten.

**Verfahren 6**

725 mg (1 mmol) Nigericin werden in 10 ml Carbonylverbindung gelöst und mit 200 mg Zinkchlorid unter Rückfluß erhitzt. Anschließend wird bis zur Trockne eingedampft. Der Rückstand wird in 20 ml Ethylacetat aufgenommen und mit 2 x 10 ml Wasser gewaschen. Nach dem Einrotieren wird das Ketal als fester Rückstand erhalten.

**Verfahren 7**

1 mmol Nigericinderivat wird in Essigester (20 ml) gelöst und mit einer Hydroxidlösung des entsprechenden Kations (pH 12 - 14) für 10 min gerührt. Die organische Phase wird 2 mal mit Wasser gewaschen, getrocknet und das Lösungsmittel wird im Rotationsdampfer abgezogen. Die Kontrolle der Salzbildung erfolgt mit IR-Spektroskopie (COOH) $\nu$ = 1740 nm, (COONa) $\nu$ = 1600 nm.

In den nachfolgenden Tabellen sind die gemäß den vorstehend beschriebenen Verfahren 1 bis 6 synthetisierten Verbindungen aufgeführt. In Tabelle 1 sind die verschiedenen Substituenten $R^1$ und $R^2$ der Nigericinderivate zwecks Identifizierung der Verbindungen angegeben. Ebenfalls angegeben in Tabelle 1 ist das Molekulargewicht sowie die Ausgangsverbindung, die Reaktionszeit, das Herstellungsverfahren und die chemische Ausbeute. In Tabelle 2 sind ausgewählte, charakteristische analytische Daten der erhaltenen Verbindungen angegeben (C-, H-Analysen, Molmassenpeak der Natriumverbindung im Massenspektrum und $^{13}$C-NMR-Daten).

**Tabelle 1**

| Bsp. Nr. | $R^1$ | $R^2$ | Z | Mol-gewicht | Ausgangs-Verbindung aus Bsp. | Zeit/h | Verfahren | chem. Ausbeute |
|---|---|---|---|---|---|---|---|---|
| 1 | H | H | H | 725 | - | | - | - |
| 2 | H | $SO_2CH_3$ | H | 803 | 1 | 0,5 | 2 | 95 % |
| 3 | H | $\overset{O}{\overset{\|}{C}}-CH_2OC_6H_5$ | H | 859 | 1 | 12 | 1 | 85 % |
| 4 | H | $\overset{O}{\overset{\|}{C}}-CH_2CH_2OC_6H_5$ | H | 873 | 1 | 12 | 1 | 91 % |
| 5 | H | $\overset{O}{\overset{\|}{C}}-CH_3$ | H | 767 | 1 | 5 | 1 | 82 % |
| 6 | H | $\overset{O}{\overset{\|}{C}}-C_6H_5$ | H | 829 | 1 | 12 | 1 | 78 % |
| 7 | H | $\overset{O}{\overset{\|}{C}}-CH_2C_6H_5$ | H | 843 | 1 | 12 | 1 | 82 % |
| 8 | H | 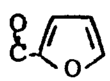 | H | 819 | 1 | 24 | 1 | 95 % |
| 9 | H | 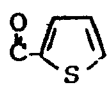 | H | 835 | 1 | 20 | 1 | 90 % |
| 10 | H | $\overset{O}{\overset{\|}{C}}-(CH_2)_{10}CH_3$ | H | 907 | 1 | 24 | 2 | 76 % |
| 11 | H | $\overset{O}{\overset{\|}{C}}-CH_2CH_2COOH$ | H | 825 | 1 | 24 | 1 | 85 % |

**Tabelle 1 Fortsetzung**

| Bsp. Nr. | $R^1$ | $R^2$ | Z | Mol-gewicht | Ausgangs-Verbindung aus Bsp. | Zeit/h | Verfahren | chem. Ausbeute |
|---|---|---|---|---|---|---|---|---|
| 12 | H | $\overset{O}{\overset{\|}{C}}$-NH-$C_6H_5$ | H | 844 | 1 | 24 | 3 | 92 % |
| 13 | H | $\overset{O}{\overset{\|}{C}}$-NH$CH_2C_6H_5$ | H | 858 | 1 | 24 | 3 | 87 % |
| 14 | H | $\overset{O}{\overset{\|}{C}}$-NH-$C_6H_4$-Cl (para) | H | 878,5 | 1 | 20 | 3 | 90 % |
| 15 | H | $SO_3H$ | H | 805 | 1 | 20 | 4 | 65 % |
| 16 | $CH_3$ | H | H | 739 | 1 | 2 | 5 | 100 % |
| 17 | $C_2H_5$ | H | H | 753 | 1 | 2 | 5 | 85 % |
| 18 | $CH_3$ | $CH_3$ | H | 765 | 1 | 2 | 6 | 95 % |
| 19 | H | $CH_3$ | H | 751 | 1 | 2 | 6 | 95 % |
| 20 | $CH_3$ | $SO_2CH_3$ | H | 817 | 2 | 2 | 5 | 85 % |
| 21 | H | 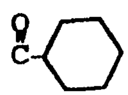 | H | 835 | 1 | 4 | 1 | 72 % |
| 22 | H | 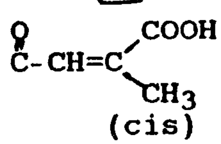 (cis) | H | 837 | 1 | 1 | 1 | 92 % |

**Tabelle 1 Fortsetzung**

| Bsp. Nr. | $R^1$ | $R^2$ | Z | Molgewicht | Ausgangs-Verbindung aus Bsp. | Zeit/h | Verfahren | chem. Ausbeute |
|---|---|---|---|---|---|---|---|---|
| 23 | H | $\overset{O}{C}$-CH=CH-COOH (cis) | H | 823 | 1 | 1 | 1 | 84 % |
| 24 | H | 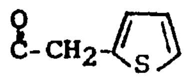 | H | 849 | 1 | 8 | 1 | 90 % |
| 25 | H | 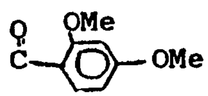 | H | 889 | 1 | 10 | 1 | 75 % |
| 26 | H | 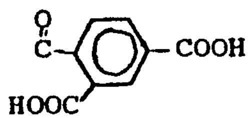 | H | 917 | 1 | 24 | 1 | 78 % |
| 27 | H | 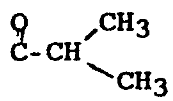 | H | 795 | 1 | 2 | 1 | 95 % |
| 28 | H | 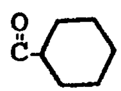 | H | 879 | 1 | 5 | 1 | 62 % |
| 29 | H | 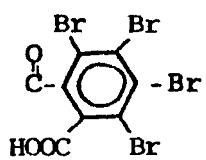 | H | 1188,7 | 1 | 10 | 1 | 75 % |
| 30 | H | $\overset{O}{C}-(CH_2)_{20}-CH_3$ | H | 1047 | 1 | 24 | 1 | 68 % |

**Tabelle 1 Fortsetzung**

| Bsp. Nr. | $R^1$ | $R^2$ | Z | Mol-gewicht | Ausgangs-Verbindung aus Bsp. | Zeit/h | Verfahren | chem. Ausbeute |
|---|---|---|---|---|---|---|---|---|
| 31 | H | 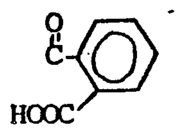 | H | 861 | 1 | 10 | 1 | 95 % |
| 32 | H | $\overset{\overset{O}{\|}}{C}-(CH_2)_3-COOH$ | H | 839 | 1 | 24 | 1 | 87 % |
| 33 | H | H | Na | 747 | 1 | 0,2 | 7 | 100 % |
| 34 | H | H | K | 763 | 1 | 0,2 | 7 | 100 % |
| 35 | H | $COCH_2CH_2COOZ$ | Na | 847 | 11 | 0,2 | 7 | 100 % |
| 36 | H | $COCH_2CH_2COOZ$ | K | 863 | 11 | 0,2 | 7 | 100 % |
| 37 | H | $COCH_2CH_2COOZ$ | Mg | 848 | 11 | 0,2 | 7 | 100 % |

**Tabelle 2**

| Bsp. Nr. | C, H ber. | | C, H gef. | | MNa (FABMS) | $^{13}C$-NMR (ppm) |
|---|---|---|---|---|---|---|
| 1 | | | | | 747 | 177,0, 107,8, 96,8, 67,8 |
| 2 | C 61,3 | H 8,8 | C 61,0 | H 8,6 | 825 | 176,4, 108,2, 95,3, 72,0 |
| 3 | C 67,1 | H 8,7 | C 66,7 | H 8,8 | 881 | |
| 4 | C 67,4 | H 8,8 | C 67,2 | H 9,1 | 895 | |
| 5 | C 65,8 | H 9,2 | C 65,6 | H 9,2 | 789 | 176,3, 171,1, 108,1, 95,8, 68,2 |
| 6 | C 68,1 | H 8,8 | C 68,4 | H 8,9 | 851 | |
| 7 | C 68,4 | H 8,8 | C 68,7 | H 8,5 | 865 | |
| 8 | C 67,3 | H 8,8 | C 67,4 | H 8,5 | 841 | |
| 9 | C 64,7 | H 8,4 | C 64,9 | H 8,9 | 857 | |
| 10 | C 68,8 | H 9,9 | C 69,1 | H 9,8 | 929 | 176,9, 173,8, 108,1, 96,1 |
| 11 | | | | | 847 | 178,7, 173,1, 172,6, 167,8, 107,8, 96,4, 67,2 |
| 12 | C 67,0 | H 8,7 | C 66,7 | H 8,4 | 867 | |
| 13 | | | | | 880 | 177,0, 153,1, 134,0, 128,3, 122,4, 118,0, 107,8, 96,7 |

**Tabelle 2 Fortsetzung**

| Bsp. Nr. | C, H ber. | | C, H gef. | | MNa (FABMS) | $^{13}C$-NMR (ppm) |
|---|---|---|---|---|---|---|
| 14 | | | | | 900 | 176,8, 153,8, 136,7, 129,0, 128,6, 119,9, 108,3, 95,8, 69,2 |
| 15 | | | | | | 178,2, 107,6, 73,2 |
| 16 | | | | | 762 | 178,7, 173,1, 172,6, 107,8, 96,4, 67,2 |
| 17 | C 67,0 | H 9,6 | C 67,3 | H 9,4 | 775 | |
| 18 | | | | | 787 | 176,4, 110,8, 107,6, 106,0 73,7 |
| 19 | C 67,2 | H 9,4 | C 67,0 | H 9,5 | 773 | |
| 20 | | | | | 839 | 177,2, 130,8, 107,3, 97,9, 69,2 |
| 21 | | | | | 857 | 176,6, 176,6, 108,1, 96,2 |
| 22 | | | | | 859 | 178,9, 176,7, 167,0, 156,6, 112,9, 107,7, 96,5 |
| 23 | | | | | 845 | 178,8, 173,9, 167,4, 142,9, 117,4, 107,8, 96,6 |
| 24 | | | | | 871 | 176,2, 170,2, 135,7, 126,7, 126,5, 124,6, 108,1, 96,0 |
| 25 | | | | | 911 | |

**Tabelle 2 Fortsetzung**

| Bsp. Nr. | C, H ber. | C, H gef. | MNa (FABMS) | $^{13}$C-NMR (ppm) |
|---|---|---|---|---|
| 26 | | | 939 | |
| 27 | | | 818 | 178,8, 108,2, 96,3 |
| 28 | | | 901 | 180,9, 178,1, 174,7, 107,5, 96,5 |
| 29 | | | 1212 | 178,2, 169,8, 164,8, 144,0, 134,2, 131,4, 127,7, 122,2, 121,9, 107,9 96,5 |
| 30 | | | 1070 | 176,3, 173,6, 108,2, 96,0 |
| 31 | | | 895 | |
| 32 | | | 862 | |

**Antivirale Wirksamkeit**

Antivirale Aktivität in der Zellkultur

Die Prüfsubstanzen wurden in Zellkulturmedium (Dulbecco's MEM) gelöst und in einer geometrischen Verdünnungsreihe, Faktor 3, in Standard-Mikrotiterplatten in 100 µl Zellkulturmedium vorgelegt. Anschließend erfolgte die Zugabe von 100 µl einer Suspension von HeLa- bzw. Vero-Zellen in Medium mit 5 % fötalem Kälberserum in einer Zelldichte von 2 x $10^5$ Zellen/ml. Die Ansätze wurden mit 50 µl einer Suspension des jeweiligen Test-Virus infiziert, die so eingestellt war, daß die Zellen innerhalb von 72 h einen cytopathogenen Effekt (CPE) zeigten. Die Auswertung erfolgte durch mikroskopische Begutachtung des Zellrasens und photometrische Messung der Neutralrotaufnahme (Farbtest nach Finter). Als MHK wurde die Konzentration des Präparats angenommen (µg/ml), bei der etwa 50 % der Zellen die Infektion überlebten.

In Tabelle 3 ist die Wirkung (Angabe des MHK in µg/ml) von verschiedenen erfindungsgemäßen Verbindungen gegen folgende Viren aufgeführt:
Adeno 5, Vaccina, Herpes I, Herpes II, Influenza A, Paramyxo. III, Rhinovirus II.

**Tabelle 3: MHK in µg/ml**

| Verb. aus Bsp. | Adeno 5 | Vaccina | Herpes I | Herpes II | Influenza A | Paramyxo. III | Rhino. II |
|---|---|---|---|---|---|---|---|
| 1 | 0,0018 | 0,049 | < 0,0018 | 0,0055 | 0,0165 | 0,0494 | 0,148 |
| 2 | < 0,02 | - | < 0,02 | < 0,02 | < 0,02 | - | < 0,02 |
| 3 | 0,002 | 1,48 | 0,006 | 0,002 | 0,49 | 1,48 | 1,48 |
| 4 | < 0,02 | < 0,02 | < 0,02 | 0,49 | 0,49 | - | 1,48 |
| 5 | < 0,02 | 0,16 | < 0,02 | < 0,02 | 0,05 | - | < 0,02 |
| 6 | 0,05 | < 0,02 | 0,05 | 0,49 | 1,48 | - | 1,48 |
| 7 | < 0,02 | < 0,02 | < 0,02 | 0,16 | 0,49 | - | 0,49 |
| 8 | < 0,02 | < 0,02 | < 0,02 | 0,49 | 0,49 | 0,49 | - |
| 9 | < 0,02 | < 0,02 | < 0,02 | 0,16 | 0,49 | - | 4,44 |
| 10 | 4,4 | - | 4,4 | 13,3 | 1,48 | - | - |
| 11 | < 0,02 | < 0,02 | < 0,02 | < 0,02 | < 0,02 | - | 0,06 |
| 12 | < 0,02 | < 0,02 | < 0,02 | 0,49 | 0,16 | - | - |
| 13 | 1,48 | - | 0,49 | 0,49 | 0,49 | - | - |
| 14 | 1,48 | 13,3 | 0,49 | 1,48 | 0,16 | 4,4 | 4,4 |
| 15 | 0,49 | 0,49 | 0,49 | 0,16 | 4,44 | 4,4 | 4,4 |
| 16 | 0,06 | 0,06 | 0,06 | < 0,02 | 0,50 | - | 1,48 |
| 17 | 0,16 | 4,4 | 0,49 | 0,05 | 1,48 | - | - |
| 18 | 0,05 | - | 0,49 | < 0,02 | - | - | 0,16 |
| 19 | 0,16 | - | 0,49 | < 0,02 | - | - | 1,48 |
| 20 | 1,48 | - | 0,49 | 0,49 | 0,16 | - | 13,3 |
| 21 | 0,02 | 0,02 | 0,02 | 0,02 | 0,02 | - | 0,49 |
| 22 | < 0,02 | < 0,02 | < 0,02 | < 0,02 | 0,49 | - | 1,48 |
| 23 | < 0,02 | < 0,02 | < 0,02 | < 0,02 | 0,05 | - | 0,16 |
| 24 | < 0,02 | < 0,02 | < 0,02 | < 0,02 | 0,16 | - | 0,16 |

Tabelle 3: MHK in µg/ml

| Verb. aus Bsp. | Adeno 5 | Vaccina | Herpes I | Herpes II | Influenza A | Paramyxo. III | Rhino. II |
|---|---|---|---|---|---|---|---|
| 25 | < 0,02 | < 0,02 | < 0,02 | < 0,02 | 0,49 | - | 0,49 |
| 26 | < 0,16 | < 0,02 | 0,16 | 0,16 | 4,44 | - | 4,44 |
| 27 | < 0,02 | < 0,02 | < 0,02 | < 0,02 | 0,49 | - | 0,16 |
| 28 | < 0,02 | < 0,02 | < 0,02 | 0,16 | 1,48 | - | 1,44 |
| 29 | 0,17 | 0,17 | 0,17 | 4,44 | 13,3 | - | 13,3 |
| 30 | 13,3 | 4,44 | 4,44 | 40,0 | 40,0 | - | - |
| 31 | < 0,02 | < 0,02 | < 0,02 | < 0,02 | 0,16 | - | 1,48 |
| 33 | < 0,02 | - | 0,002 | 0,016 | 0,44 | - | 0,41 |
| 34 | < 0,02 | - | 0,005 | 0,016 | 0,44 | - | - |
| 35 | < 0,18 | - | 0,18 | 0,004 | < 0,02 | - | < 0,02 |
| 36 | < 0,18 | - | 0,04 | 0,004 | < 0,02 | - | - |
| 37 | < 0,18 | - | 0,001 | 0,004 | < 0,02 | - | - |

**Antibakterielle Wirksamkeit**

Die antibakterielle Wirksamkeit der erfindungsgemäßen Verbindungen wurde im Agar-Verdünnungstest nach Lorian (Antibiotics in Laboratory Medicine, Williams & Wilkins, Baltimore/London, 1980) bestimmt. Dabei wurden die Präparate in einer geometrischen Verdünnungsreihe mit dem Faktor 2 in Müller Hinton Agar verdünnt. Eine Petrischale enthielt nur Müller Hinton Agar und diente zur Kontrolle des Bakterienwachstums. Die Petrischalen wurden dann mit einem Denley-Multipoint-Inokulator, welcher 0,6 µl einer 1 : 100 verdünnten 18-Stunden-Kultur der Testbakterien übertrug, mit den entsprechenden Testbakterien beimpft. Nach 16 bis 18 Stunden Inkubation bei 37° C wurden die Petrischalen makroskopisch auf Bakterienwachstum untersucht.

Die niedrigste Konzentration der erfindungsgemäßen Verbindungen, welche das Bakterienwachstum noch vollständig verhinderte, wurde als MIC (Minimum Inhibitory Concentration) angenommen.

In Tabelle 4 ist die Wirkung (Angabe des MIC in µg/ml) von verschiedenen erfindungsgemäßen Verbindungen gegen folgende Bakterien aufgeführt:
Staph. aureus (SG 511), Staph. aureus (285), Staph. aureus (503), Strept. pyogenes (308 A), Strept pyogenes (77 A), Strept. faecium (D).

Nigericin (Verbindung A) diente als Vergleichssubstanz.

Überraschend zeigen die Dicarbonsäure-Addukte des Nigericins auch eine Wirkung gegen gramnegative Bakterien. So besitzt beispielsweise die Substanz aus Beispiel 32 gegen E. coli eine MHK von 0,55 µg/ml, gegen E. coli (DC 2) eine MHK von 25,0 µg/ml und gegen Entrob. Cloacae (1321 E) eine MHK von 250 µg/ml.

**Tabelle 4: MIC in µg/ml**

| Verb. aus Bsp. | Staph. aureus (SG 511) | Staph. aureus (285) | Staph. aureus (503) | Strept. pyogenes (308 A) | Strept. pyogenes (77 A) | Strept. faecium (D) |
|---|---|---|---|---|---|---|
| 1 | 0,098 | 0,098 | 0,098 | 0,025 | 0,013 | 0,195 |
| 2 | 0,098 | 0,049 | 0,049 | 0,004 | 0,013 | 0,195 |
| 3 | 0,391 | 0,391 | 0,391 | 0,049 | 0,049 | 0,781 |
| 4 | 0,78 | 1,56 | 0,78 | 0,098 | 0,098 | 1,56 |
| 5 | 0,391 | 0,391 | 0,391 | 0,098 | 0,098 | 0,781 |
| 6 | 0,098 | 0,391 | 0,098 | 0,013 | 0,013 | 0,391 |
| 7 | 0,195 | 0,391 | 0,195 | 0,004 | 0,004 | 0,391 |
| 8 | 0,195 | 0,391 | 0,195 | 0,013 | 0,013 | 0,391 |
| 9 | 0,049 | 0,195 | 0,049 | 0,007 | 0,007 | 0,195 |
| 10 | 6,25 | 6,25 | 3,13 | 0,781 | 0,781 | 6,25 |
| 11 | 0,391 | 0,195 | 0,098 | 0,025 | 0,025 | 0,391 |
| 12 | 0,781 | 3,13 | 0,78 | 0,098 | 0,098 | 1,56 |
| 13 | 3,13 | 6,25 | 3,13 | 0,78 | 0,39 | 12,5 |
| 14 | 100,0 | 100,0 | 50,0 | 25,0 | 12,5 | 100,0 |
| 15 | 1,56 | 1,56 | 0,78 | 1,56 | 0,78 | 3,13 |
| 16 | 1,56 | 1,56 | 1,56 | 0,39 | 0,39 | 3,13 |

**Tabelle 4: Fortsetzung**

| Verb. aus Bsp. | Staph. aureus (SG 511) | Staph. aureus (285) | Staph. aureus (503) | Strept. pyogenes (308 A) | Strept. pyogenes (77 A) | Strept. faecium (D) |
|---|---|---|---|---|---|---|
| 17 | 1,56 | 1,56 | 0,78 | 0,098 | 0,098 | 1,56 |
| 18 | 6,25 | 6,25 | 3,13 | 0,391 | 1,56 | 6,25 |
| 19 | 12,5 | 12,5 | 6,25 | 1,56 | 1,56 | 12,5 |
| 20 | 25,0 | 25,0 | 12,5 | 6,25 | 6,25 | 25,0 |
| 21 | 0,195 | 0,391 | 0,195 | 0,004 | 0,004 | 0,391 |
| 22 | 6,25 | 6,25 | 6,25 | 0,78 | 0,78 | 6,25 |
| 23 | 0,195 | 0,391 | 0,195 | 0,013 | 0,025 | 0,39 |
| 24 | 0,049 | 0,098 | 0,049 | 0,004 | 0,004 | 0,098 |
| 25 | 1,56 | 1,56 | 0,78 | 0,098 | 0,195 | 1,56 |
| 26 | 6,25 | 6,25 | 6,25 | 1,56 | 1,56 | > 100 |
| 27 | 0,39 | 0,39 | 0,19 | 0,049 | 0,049 | > 100 |
| 28 | 1,56 | 1,56 | 1,56 | 0,195 | 0,195 | 1,56 |
| 29 | 1,56 | 3,13 | 1,56 | 0,195 | 0,195 | 3,13 |
| 30 | 100,0 | 100,0 | 50,0 | 25,0 | 25,0 | 100,0 |
| 31 | 0,78 | 0,78 | 0,78 | 0,025 | 0,025 | 0,781 |
| 32 | 0,39 | 0,39 | 0,39 | 0,098 | 0,098 | 0,39 |
| 33 | 0,049 | 0,049 | 0,049 | 0,004 | 0,007 | 0,195 |

**Tabelle 4: Fortsetzung**

| Verb. aus Bsp. | Staph. aureus (SG 511) | Staph. aureus (285) | Staph. aureus (503) | Strept. pyogenes (308 A) | Strept. pyogenes (77 A) | Strept. faecium (D) |
|---|---|---|---|---|---|---|
| 34 | 0,025 | 0,025 | < 0,002 | < 0,002 | < 0,002 | 0,098 |
| 35 | 0,025 | 0,025 | 0,013 | < 0,002 | < 0,002 | 0,049 |
| 36 | 0,025 | 0,025 | 0,013 | < 0,002 | < 0,002 | 0,049 |
| 37 | 0,098 | 0,098 | 0,049 | 0,007 | 0,007 | 0,098 |

## Ansprüche

1. Nigericinderivate der Formel I

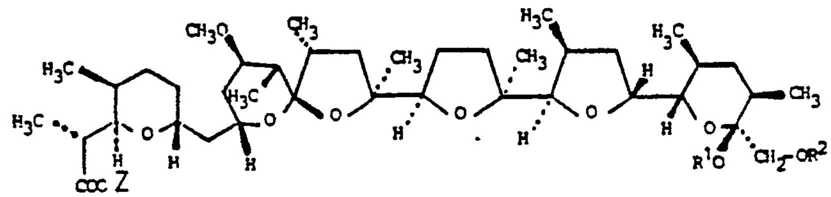

(I)

in der
Z Wasserstoff, Na, K, Li, Mg, Ca, $NH_4$ bedeutet, und
R' Wasserstoff oder $C_1$-$C_8$-Alkyl bedeutet und
$R^2$ Wasserstoff bedeutet oder einen Rest der Formel
$-\overset{O}{\overset{\|}{C}}-R^3$, $-\overset{O}{\overset{\|}{C}}-NH-R^3$ oder $-\overset{O}{\overset{\|}{C}}-O-R^3$ darstellt, in der $R^3$ $C_1$-$C_{26}$-Alkyl, $C_2$-$C_{15}$-Alkenyl oder $C_3$-$C_9$-Cycloalkyl bedeutet, wobei diese Alkyl-, Alkenyl- und Cycloalkylreste gegebenenfalls halogen-, nitro-, cyano- carboxyl-, $C_1$-$C_4$-alkoxy-, thienyl-, phenyloxy- oder phenylsubstituient sind, wobei die genannten Phenylreste ihrerseits mit Halogen, Nitro, Cyano, $C_1$-$C_4$-Alkyl oder $C_1$-$C_4$-Alkoxy substituiert sein können, oder in der
$R^3$ Phenyl, Furyl oder Thienyl bedeutet, wobei diese Phenyl-, Furyl- oder Thienyl-Reste mit Halogen, Nitro, Cyano, Carboxyl-, $C_1$-$C_4$-Alkyl oder $C_1$-$C_4$-Alkoxy substituiert sein können
oder worin
$R^2$ einen Sulfonsäureester der Formel $-SO_2R^4$ darstellt, worin
$R^4$ Hydroxy, $C_1$-$C_6$-Alkyl, Phenyl oder p-Toloyl bedeutet
oder worin
$R^1$ und $R^2$ zusammen einen Rest der Formel II darstellen

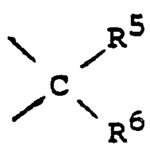

(II)

in der
$R^5$ und $R^6$ gleich oder verschieden sind und Wasserstoff oder $C_1$-$C_{10}$-Alkyl bedeuten oder wobei
$R^5$ und $R^6$ eine Alkylenkette darstellen, die zusammen mit dem sie tragenden C-Atom einen 5-, 6- oder 7-gliedrigen Ring bildet,
ausgenommen die Verbindungen der Formel I,
in der $R^1$ und $R^2$ gleichzeitig Wasserstoff bedeuten (Nigericin) und solche, in der $R^1$ Wasserstoff und $R^2$ Acetyl oder Benzoyl bedueten und das Natriumsalz der Verbindung der Formel I, in der $R^1$ und $R^2$ zusammen einen Rest der Formel II darstellen, mit $R^5$ = $R^6$ = $CH_3$.

2. Nigericinderivate nach Anspruch 1, dadurch gekennzeichnet, daß mindestens eine der nachfolgenden Bedingungen erfüllt ist:
Z bedeutet Wasserstoff, Na, K, Mg, Ca, $NH_4$,
$R^1$ bedeutet Wasserstoff oder $C_1$-$C_4$-Alkyl;
$R^3$ bedeutet $C_1$-$C_{22}$-Alkyl, $C_2$-$C_8$-Alkenyl oder $C_5$-$C_7$-Cycloalkyl, wobei diese Alkyl-, Alkenyl- und Cycloalkylreste gegebenenfalls carboxyl-, thienyl-, phenyloxy- oder phenylsubstituiert sind, wobei die genannten Phenylreste ihrerseits mit Halogen substituiert sein können, oder
$R^3$ bedeutet Phenyl, Furyl oder Thienyl, wobei diese Phenyl-, Furyl- oder Thienylreste mit Halogen, Carboxyl- oder $C_1$-$C_4$-Alkoxy substituiert sein können,
$R^4$ bedeutet Hydroxy oder $C_1$-$C_4$-Alkyl,
$R^5$ und $R^6$ sind gleich oder verschieden und bedeuten Wasserstoff oder $C_1$-$C_4$-Alkyl.

3. Nigericinderivate nach Anspruch 1 und/oder 2, dadurch gekennzeichnet, daß mindestens eine der nachfolgenden Bedingungen erfüllt ist:
Z bedeutet Wasserstoff, Na, K, $NH_4$,

$R^1$ bedeutet Wasserstoff, Methyl oder Ethyl,

$R^3$ bedeutet $C_1$-$C_{22}$-Alkyl, $C_2$-$C_4$-Alkenyl oder Cyclohexyl, wobei diese Alkyl-, Alkenyl- und Cycloalkylreste gegebenenfalls carboxyl-, thienyl-, phenyloxy- oder phenylsubstituiert sind,

oder

$R^3$ bedeutet Phenyl, Furyl oder Thienyl, wobei der Phenylreste mit Chlor, Brom, Carboxyl und/oder Methoxy substituiert sein kann,

$R^4$ bedeutet Hydroxy oder Methyl,

$R^5$ und $R^6$ sind gleich oder verschieden und bedeuten Wasserstoff oder Methyl.

4. Verbindungen nach einem oder mehreren der Ansprüche 1 bis 3 zur Anwendung als Arzneimittel.

5. Verbindungen nach einem oder mehreren der Ansprüche 1 bis 3 zur Anwendung als antibakteriell wirkende Arzneimittel.

6. Verbindungen nach einem oder mehreren der Ansprüche 1 bis 3 einschließlich Nigericin und der Nigericinderivate der Formel I, in der $R^1$ Wasserstoff und $R^2$ Acetyl oder Benzoyl bedeuten und das Natriumsalz der Verbindungen der Formel I, in der $R^1$ und $R^2$ zusammen einen Rest der Formel II darstellen, mit $R^5 = R^6 = CH_3$ zur Anwendung als antiviral wirksame Arzneimittel.

7. Verfahren zur Herstellung von Verbindungen der Formel I, dadurch gekennzeichnet, daß man

a) Nigericin (Formel I : $R^1 = R^2 = H$) oder ein $R^1$-Derivat umsetzt mit einer Verbindung der Formel III oder IV

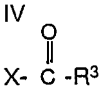 (III)

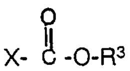 (IV)

worin

X Chlor, Brom oder ein Anhydrid bedeutet und $R^3$ die Anspruch 1 zu Formel I angegebenen Bedeutungen hat,

oder daß man

b) Nigericin (Formel I : $R^1 = R^2 = H$) oder ein $R^1$-Derivat umsetzt mit einem Isocyanat der Formel V

$O = C = N - R^3$ (V)

worin $R^3$ die in Anspruch 1 zu Formel I angegebenen Bedeutungen hat,

oder daß man

c) Nigericin (Formel I : $R^1 = R^2 = H$) oder ein $R^1$-Derivat umsetzt mit einem $SO_3$/Pyridin-Komplex oder mit einer Verbindung der Formel Y-$SO_2$-$R^4$, wobei Y Chlor oder Brom bedeutet und $R^4$ bis auf Wasserstoff, die in Anspruch 1 zu Formel I angegebenen Bedeutungen hat,

oder daß man

d) Nigericin (Formel I : $R^1 = R^2 = H$) oder ein $R^2$-Derivat in Gegenwart einer Lewis-Säure umsetzt mit einem Alkohol der Formel VI

$R^1 - OH$ (VI)

worin $R^1$ bis auf Wasserstoff die in Anspruch 1 zu Formel I angegebenen Bedeutungen hat,

oder daß man

e) Nigericin (Formel I : $R^1 = R^2 = H$) in Gegenwart eines Katalysators umsetzt mit einer Carbonylverbindung der Formel VII

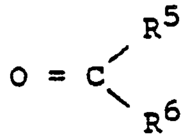 (VII)

worin $R^5$ und $R^6$ die in Anspruch 1 zu Formel I angegebenen Bedeutungen haben, und wobei man gegebenenfalls anschließend die Verbindungen der Formel I in ihr physiologisch verträgliches Salz überführt.

8. Arzneimittel, enthaltend eine Verbindung gemäß Anspruch 1, 2 oder 3.

9. Verfahren zur Herstellung von Arzneimitteln, welche antiviral wirken, dadurch gekennzeichnet, daß man in das Arzneimittel eine Verbindung gemäß Anspruch 6 einverleibt.

10. Verfahren zur Herstellung von Arzneimitteln, welche antibakteriell wirken, dadurch gekennzeichnet, daß man in das Arzneimittel eine Verbindung gemäß Anspruch 1, 2 oder 3 einverleibt.

Patentansprüche für folgende Vertragsstaaten: ES, GR

1. Verfahren zum Herstellen einer Verbindung I

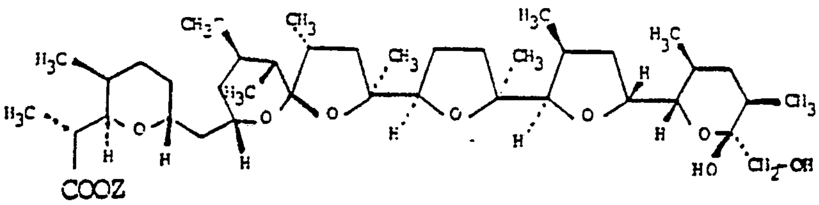

(I)

in der
Z Wasserstoff, Na, K, Li, Mg, Ca, $NH_4$ bedeutet, und
$R^1$ Wasserstoff oder $C_1$-$C_8$-Alkyl bedeutet und
$R^2$ Wasserstoff bedeutet oder einen Rest der Formel
$-\overset{O}{\overset{\|}{C}}-R^3$, $-\overset{O}{\overset{\|}{C}}-NH-R^3$ oder $-\overset{O}{\overset{\|}{C}}-O-R^3$ darstellt, in der $R^3$ $C_1$-$C_{26}$-Alkyl, $C_2$-$C_{15}$-Alkenyl oder $C_3$-$C_9$-Cycloalkyl bedeutet, wobei diese Alkyl-, Alkenyl- und Cycloalkylreste gegebenenfalls halogen-, nitro-, cyano- carboxyl-, $C_1$-$C_4$-alkoxy-, thienyl-, phenyloxy- oder phenylsubstituiert sind, wobei die genannten Phenylreste ihrerseits mit Halogen, Nitro, Cyano, $C_1$-$C_4$-Alkyl oder $C_1$-$C_4$-Alkoxy substituiert sein können, oder in der
$R^3$ Phenyl, Furyl oder Thienyl bedeutet, wobei diese Phenyl-, Furyl- oder Thienyl-Reste mit Halogen, Nitro, Cyano, Carboxyl-, $C_1$-$C_4$-Alkyl oder $C_1$-$C_4$-Alkoxy substituiert sein können
oder worin
$R^2$ einen Sulfonsäureester der Formel $-SO_2R^4$ darstellt, worin
$R^4$ Hydroxy, $C_1$-$C_6$-Alkyl, Phenyl oder p-Toloyl bedeutet
oder worin
$R^1$ und $R^2$ zusammen einen Rest der Formel II darstellen

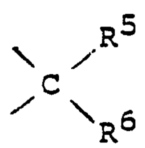

(II)

in der
$R^5$ und $R^6$ gleich oder verschieden sind und Wasserstoff oder $C_1$-$C_{10}$-Alkyl bedeuten oder wobei
$R^5$ und $R^6$ eine Alkylenkette darstellen, die zusammen mit dem sie tragenden C-Atom einen 5-, 6- oder 7-gliedrigen Ring bildet,
ausgenommen die Verbindungen der Formel I,
in der $R^1$ und $R^2$ gleichzeitig Wasserstoff bedeuten (Nigericin) und solche, in der $R^1$ Wasserstoff und $R^2$ Acetyl oder Benzoyl bedueten und das Natriumsalz der Verbindung der Formel I, in der $R^1$ und $R^2$ zusammen einen Rest der Formel II darstellen, mit $R^5 = R^6 = CH_3$,
dadurch gekennzeichnet, daß man
a) Nigericin (Formel I : $R^1 = R^2 = H$) oder ein $R^1$-Derivat umsetzt mit einer Verbindung der Formel III oder IV

$X-\overset{O}{\overset{\|}{C}}-R^3$ (III)

$X-\overset{O}{\overset{\|}{C}}-O-R^3$ (IV)

worin
X Chlor, Brom oder ein Anhydrid bedeutet und
$R^3$ die Anspruch 1 zu Formel I angegebenen Bedeutungen hat,

oder daß man
b) Nigericin (Formel I : $R^1 = R^2 = H$) oder ein $R^1$-Derivat umsetzt mit einem Isocyanat der Formel V
$O = C = N - R^3$ (V)
worin $R^3$ die in Anspruch 1 zu Formel I angegebenen Bedeutungen hat,
oder daß man
c) Nigericin (Formel I : $R^1 = R^3 = H$) oder ein $R^1$-Derivat umsetzt mit einem $SO_3$/Pyridin-Komplex oder mit einer Verbindung der Formel $Y\text{-}SO_2\text{-}R^4$, wobei Y Chlor oder Brom bedeutet und $R^4$ bis auf Wasserstoff, die in Anspruch 1 zu Formel I angegebenen Bedeutungen hat,
oder daß man
d) Nigericin (Formel I : $R^1 = R^2 = H$) oder ein $R^2$-Derivat in Gegenwart einer Lewis-Säure umsetzt mit einem Alkohol der Formel VI
$R^1 - OH$ (VI)
worin $R^1$ bis auf Wasserstoff die in Anspruch 1 zu Formel I angegebenen Bedeutungen hat,
oder daß man
e) Nigericin (Formel I : $R^1 = R^2 = H$) in Gegenwart eines Katalysators umsetzt mit einer Carbonylverbindung der Formel VII

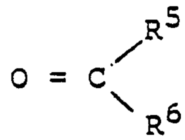

(VII)

worin $R^5$ und $R^6$ die in Anspruch 1 zu Formel I angegebenen Bedeutungen haben,
und wobei man gegebenenfalls anschließend die Verbindungen der Formel I in ihr physiologisch verträgliches Salz überführt.

2. Verfahren nach Anspruch 1, dadurch gekennzeichnet, daß mindestens eine der nachfolgenden Bedingungen erfüllt ist:
Z bedeutet Wasserstoff, Na, K, Mg, Ca, $NH_4$,
$R^1$ bedeutet Wasserstoff oder $C_1$-$C_4$-Alkyl;
$R^3$ bedeutet $C_1$-$C_{22}$-Alkyl, $C_2$-$C_8$-Alkenyl oder $C_5$-$C_7$-Cycloalkyl, wobei diese Alkyl-, Alkenyl- und Cycloalkylreste gegebenenfalls carboxyl-, thienyl-, phenyloxy- oder phenylsubstituiert sind, wobei die genannten Phenylreste ihrerseits mit Halogen substituiert sein können, oder
$R^3$ bedeutet Phenyl, Furyl oder Thienyl, wobei diese Phenyl-, Furyl- oder Thienylreste mit Halogen, Carboxyl- oder $C_1$-$C_4$-Alkoxy substituiert sein können,
$R^4$ bedeutet Hydroxy oder $C_1$-$C_4$-Alkyl,
$R^5$ und $R^6$ sind gleich oder verschieden und bedeuten Wasserstoff oder $C_1$-$C_4$-Alkyl.

3. Verfahren nach Anspruch 1, dadurch gekennzeichnet, daß mindestens eine der nachfolgenden Bedingungen erfüllt ist:
Z bedeutet Wasserstoff, Na, K, $NH_4$,
$R^1$ bedeutet Wasserstoff, Methyl oder Ethyl,
$R^3$ bedeutet $C_1$-$C_{22}$-Alkyl, $C_2$-$C_4$-Alkenyl oder Cyclohexyl, wobei diese Alkyl-, Alkenyl- und Cycloalkylreste gegebenenfalls carboxyl-, thienyl-, phenyloxy- oder phenylsubstituiert sind,
oder
$R^3$ bedeutet Phenyl, Furyl oder Thienyl, wobei der Phenylreste mit Chlor, Brom, Carboxyl und/oder Methoxy substituiert sein kann,
$R^4$ bedeutet Hydroxy oder Methyl,
$R^5$ und $R^6$ sind gleich oder verschieden und bedeuten Wasserstoff oder Methyl.

4. Verfahren zur Herstellung von Arzneimitteln, welche antibakteriell wirken, dadurch gekennzeichnet, daß man in das Arzneimittel eine Verbindung gemäß Anspruch 1, 2 oder 3 einverleibt.